# EUROPEAN PATENT APPLICATION

(11) **EP 2 959 827 A1**
(43) Date of publication of application: **30.12.2015**
(21) Application number: 15168937.9
(22) Date of filing: 22.05.2015
(51) Int. Cl.: A61B 5/00, A61B 5/11, A61B 5/08, A61B 5/0245, A61B 7/00

(54) **SLEEP STATE ESTIMATION DEVICE, METHOD AND STORAGE MEDIUM**

(30) Priority: 25.06.2014 JP 2014130291
(71) Applicant: Kabushiki Kaisha Toshiba, Minato-ku Tokyo 105-8001 (JP)
(72) Inventor: Takakura, Junya, Tokyo 105-8001 (JP); Nakayama, Kanako, Tokyo 105-8001 (JP); Yamauchi, Yasunobu, TTokyo 105-8001 (JP)
(74) Representative: Noble, Nicholas

(57) **Abstract**

According to one embodiment, a sleep state estimation device (10) attached to a user during sleep and used is provided. The device includes a first detector (111), a first estimation module (112), a second detector (113), and a second estimation module (114). The first detector is configured to detect a first signal to estimate a first state of the user. The first estimation module is configured to estimate the user's first state, based on the first signal. The second detector is configured to detect a second signal to estimate a second state of the user other than the user's first state. The second estimation module is configured to estimate the user's second state by using the second signal, based on an estimation method determined in accordance with the first signal.

## Description

### FIELD

Embodiments described herein relate generally to a sleep state estimation device, a method and a storage medium.

### BACKGROUND

In general, a user's state during sleep (hereinafter referred to as a sleep state) is estimated by using a body position sensor, a snoring sensor, etc., for diagnosis of, for example, sleep apnea syndrome, etc.

In this case, the body sensor is attached to, for example, the user's chest, etc., and the snoring sensor is attached to, for example, the user's throat, etc. Sleeping with a plurality of sensors separately attached at different points is very troublesome for the user.

Recently, a sleep state estimation device (sleep sensor) including a plurality of sensors used to estimate the user's sleep state has been developed. According to such a sleep state estimation device, not only the user's body position and respiratory state during sleep, but also other conditions such as electrocardiographic activity, pulse and body temperature can be estimated. In addition, the inconvenience for the user can be reduced since separately attaching a plurality of sensors as mentioned above is unnecessary.

The above-explained sleep state estimation device is capable of estimating various states as the user's sleep state but, the estimation accuracy may be low since each state is estimated by using a signal alone detected by the sensor corresponding to the state.

### BRIEF DESCRIPTION OF THE DRAWINGS

FIG. 1 is an illustration showing an example of use of a sleep state estimation device of a first embodiment.
FIG. 2 is a plan view showing an example of an attachment surface of the sleep state estimation device.
FIG. 3 is an illustration showing an example of attachment of the sleep state estimation device.
FIG. 4 is a diagram showing an example of a system configuration of the sleep state estimation device.
FIG. 5 is a diagram showing an example of a structure of an electrocardiographic sensor module shown in FIG. 4.
FIG. 6 is a diagram showing an example of a structure of an acceleration sensor module shown in FIG. 4.
FIG. 7 is a block diagram mainly showing an example of a functional structure of the sleep state estimation device of the first embodiment.
FIG. 8 is a flowchart showing an example of a procedure of body position estimation processing.
FIG. 9 is a flowchart showing an example of a procedure of respiratory state estimation processing.
FIG. 10 is a block diagram mainly showing an example of a functional structure of a sleep state estimation device of a second embodiment.
FIG. 11 is a flowchart showing an example of a procedure of heart rate estimation processing.
FIG. 12 is a diagram showing an example of a system configuration of a sleep state estimation device of a third embodiment.
FIG. 13 is a block diagram mainly showing an example of a functional structure of the sleep state estimation device of the third embodiment.
FIG. 14 is a flowchart showing an example of a procedure of respiratory state estimation processing in the third embodiment.

### DETAILED DESCRIPTION

Various embodiments will be described hereinafter with reference to the accompanying drawings.

In general, according to one embodiment, a sleep state estimation device attached to a user during sleep and used is provided. The device includes a first detector, a first estimation module, a second detector, and a second estimation module. The first detector is configured to detect a first signal to estimate a first state of the user. The first estimation module is configured to estimate the user's first state, based on the first signal. The second detector is configured to detect a second signal to estimate a second state of the user other than the user's first state. The second estimation module is configured to estimate the user's second state by using the second signal, based on an estimation method determined in accordance with the first signal.

### (First Embodiment)

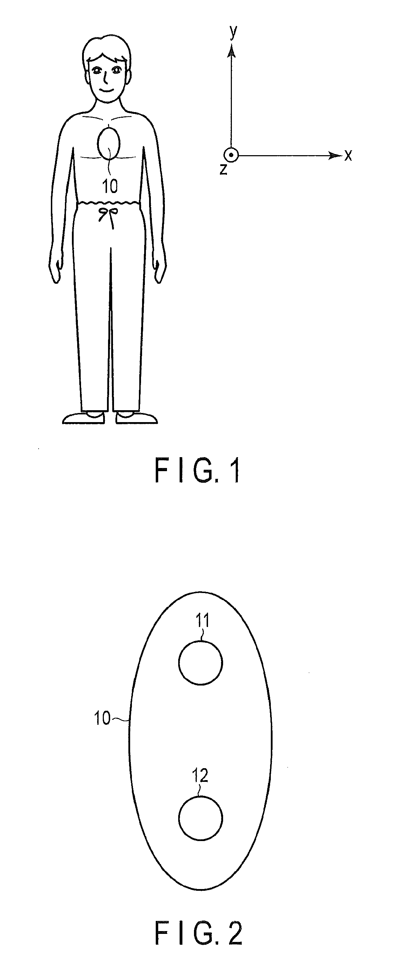

FIG. 1 shows an example of use of a sleep state estimation device of the First Embodiment. The sleep state estimation device 10 shown in FIG. 1 is a small, lightweight and thin device employed to estimate a user's state during sleep (hereinafter called a sleep state), and is used by attaching an attachment surface of the sleep state estimation device 10 on a chest, etc., of the user during sleep.

In the present embodiment, the user's sleep state estimated by the sleep state estimation device 10 indicates, for example, a heart rate, a body position (position and attitude of body), a respiratory state, etc., of the user during sleep.

In the following explanations, axes horizontal to the attachment surface and orthogonal to each other are called an x-axis and a y-axis, and an axis orthogonal to the x-axis and the y-axis (i.e., an axis normal to the attachment surface) is called a z-axis, relative to the attachment of the sleep state estimation device 10. In this case, the sleep state estimation device 10 is assumed to be attached such that the x-axis corresponds to an axis in a lateral direction of the user's body, the y-axis corresponds to an axis in a vertical direction of the user's body, and the z-axis corresponds to an axis in a front and back direction of the user, as shown in FIG. 1.

FIG. 2 is a plan view showing an example of the attachment surface (i.e., back surface) of the sleep state estimation device 10. The sleep state estimation device 10 is, for example, in an ellipsoidal shape (or a substantially rectangular shape) having a longitudinal axis of approximately several centimeters, and two electrocardiographic electrodes 11 and 12 are arranged on the attachment surface of the sleep state estimation device 10 as shown in FIG. 2. The electrocardiographic electrodes 11 and 12 are spaced apart (arranged in close vicinity of both ends) along the longitudinal axis of the attachment surface of the sleep state estimation device 10.

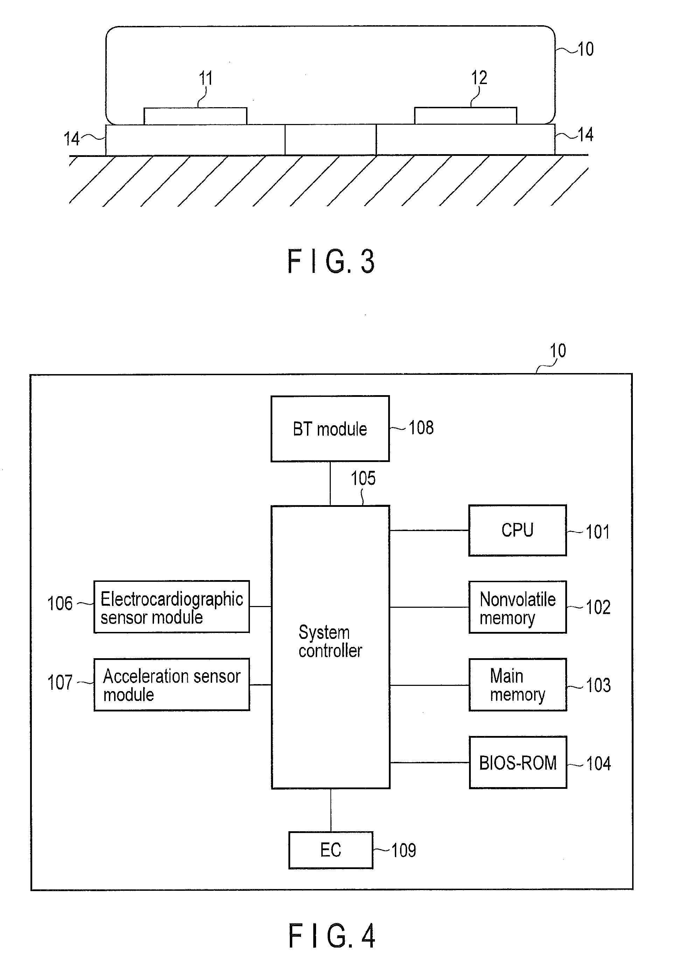

The attachment surface of the sleep state estimation device 10 contacts (skin of) the user via an electrically conductive gel 14 arranged at a position in contact with each of the electrocardiographic electrodes 11 and 12, as shown in FIG. 3. The electrically conductive gel (material) 14 may be adhesive or the sleep state estimation device 10 may be attached to the user with a double-sided tape, a belt or the like. The gel 14 is thick and elastic.

FIG. 4 is a diagram showing a system configuration of the sleep state estimation device 10. The sleep state estimation device 10 includes a CPU 101, a nonvolatile memory 102, a main memory 103, a BIOS-ROM 104, a system controller 105, an electrocardiographic sensor module 106, an acceleration sensor module 107, a BT module 108, an EC 109, etc., as shown in FIG. 4.

The CPU 101 is a processor configured to control operations of respective components in the sleep state estimation device 10. The CPU 101 executes various types of software loaded from the nonvolatile memory 102, which is a storage device, to the main memory 103.

The CPU 101 also executes a Basic Input/Output System (BIOS) stored in the BIOS-ROM 104. The BIOS is a program for hardware control.

The system controller 105 is a bridge device which makes connection between the CPU 101 and various components. The CPU 101, the nonvolatile memory 102, the main memory 103, the BIOS-ROM 104, the electrocardiographic sensor module 106, the acceleration sensor module 107, the BT module 108, the EC 109, etc., are connected to the system controller 105.

The electrocardiographic sensor module 106 is a module which includes an electrocardiographic sensor capable of sensing an electrocardiographic signal and which is employed to estimate the user's heart rate during sleep. The acceleration sensor module 107 is a module which includes an acceleration sensor capable of sensing an acceleration signal and which is employed to estimate the user's body position and respiratory state during sleep. Structures of the electrocardiographic sensor module 106 and the acceleration sensor module 107 will be described later.

The BT module 108 is a module configured to execute wireless communication of Bluetooth(TM) with a Bluetooth-enabled device. The Bluetooth-enabled device implies, for example, a smartphone, a tablet computer, a personal computer (PC) or the like.

The EC 109 is an electric power management controller which executes electric power management of the sleep state estimation device 10.

The sleep state estimation device 10 of the present embodiment may includes a plurality of types of sensors capable of sensing a pulse wave, a body temperature, etc., besides the electrocardiographic sensor and the acceleration sensor, though not shown in FIG. 2.

The sleep state estimation device 10 thus includes a plurality of sensors in a housing but, since analog front-ends of the plurality of sensors are different in specification for the respective sensors, the device is required to have both flexibility and high performance and may be upsized. In the present embodiment, however, a module which is several millimeters square is implemented by integrating a plurality of analog front-ends, the CPU 101, etc., on a single chip by pseudo-SoC technology. The pseudo-SoC technology establishes compatibility of miniaturization corresponding to SoC and degree of freedom corresponding to SiP by integrating components on a wafer. The small, lightweight (ten grams or so) and thin (several millimeters or so) sleep state estimation device 10 can be implemented by connecting a small number of peripheral components such as an antenna and a battery to the module. Implementation of miniaturization of the sleep state estimation device 10 using pseudo-SoC technology is explained here, but the miniaturization can be implemented with, for example, an LSI, etc.

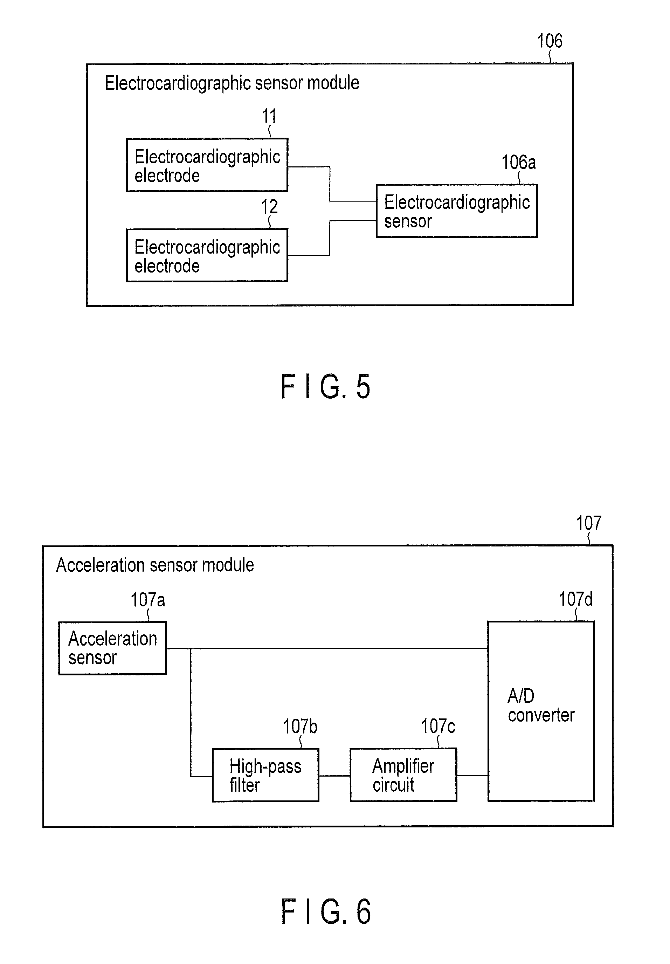

FIG. 5 shows a structure of the electrocardiographic sensor module 106 shown in FIG. 4. In the electrocardiographic sensor module 106, the electrocardiographic electrodes 11 and 12 are connected to an electrocardiographic sensor 106a which is the analog front-end for electrocardiogram, as shown in FIG. 5.

The electrocardiographic sensor 106a is a sensor which senses an electrocardiographic signal indicating an electrocardiographic state of the user to which the sleep state estimation device 10 is attached. The electrocardiographic signal sensed by the electrocardiographic sensor 106a includes, for example, a time-series signal obtained by sampling a potential difference between the electrocardiographic electrodes 11 and 12. According to the electrocardiographic signal, an electrocardiogram (electrocardiographic waveform), etc., can be obtained.

FIG. 6 shows a structure of the acceleration sensor module 107 shown in FIG. 4. The acceleration sensor module 107 includes an acceleration sensor 107a, a high-pass filter 107b, an amplifier circuit 107c, an A/D converter 107d, etc., as shown in FIG. 6.

The acceleration sensor 107a is a sensor which senses an acceleration signal indicating acceleration which acts on the sleep state estimation device 10. The acceleration sensor 107a is assumed to be, for example, a triaxial acceleration sensor (three-dimensional acceleration sensor) capable of sensing an acceleration signal of each axial direction of three orthogonal axes (i.e., the above-described x-, y- and z-axes). In addition, the acceleration signal sensed (output) by the acceleration sensor 107a is an analog signal.

The sleep state estimation device 10 is shaped in, for example, an ellipsoid as explained above, but (the acceleration signal produced according to) vibration can hardly be sensed in vicinity of a center of the sleep state estimation device 10. The acceleration sensor 107a is therefore located at, for example, a position displaced from the center of the sleep state estimation device 10 to detect the acceleration signal more correctly.

The high-pass filter 107b extracts an alternating-current component of the acceleration signal from the acceleration signal sensed by the acceleration sensor 107a.

The amplifier circuit (analog circuit) 107c amplifies the alternating-current component extracted by the high-pass filter 107b.

The A/D converter 107d converts the acceleration signal (analog signal) sensed by the acceleration sensor 107a into a digital signal. In addition, the A/D converter 107d converts the alternating-current component of the acceleration signal amplified by the amplifier circuit 107c into a digital signal.

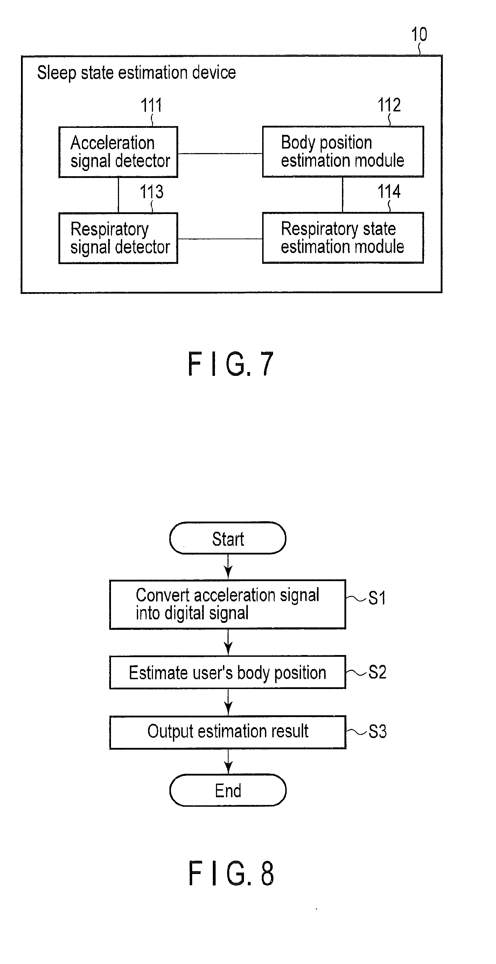

FIG. 7 is a block diagram mainly showing a functional configuration of the sleep state estimation device 10 of the present embodiment. The sleep state estimation device 10 includes an acceleration signal detector 111, a body position estimation module 112, a respiratory signal detector 113, and a respiratory state estimation module 114 as shown in FIG. 7.

The acceleration signal detector 111 is a functional module implemented by the acceleration sensor 107a, which detects the acceleration signal by the acceleration sensor 107a as a signal (first signal) to estimate the user's body position (first state). The acceleration sensor 107a of the present embodiment is a triaxial acceleration sensor as explained above. For this reason, the acceleration signal detector 111 detects the acceleration signal representing the acceleration in each axial direction of the orthogonal x-, y- and z-axes.

Each of the acceleration signals detected by the acceleration signal detector 111 includes a direct-current component mainly representing a gravitational acceleration, and the alternating-current component mainly representing the acceleration produced by the user's conditions (for example, respiratory state).

In the following explanations, an acceleration signal (first acceleration signal) indicating the acceleration of the x-axis (first axis) direction, of the acceleration signals detected by the acceleration signal detector 111, is called an x-axis acceleration signal, an acceleration signal (second acceleration signal) indicating the acceleration of the y-axis (second axis) direction is called a y-axis acceleration signal, and an acceleration signal (third acceleration signal) indicating the acceleration of the z-axis (third axis) direction is called a z-axis acceleration signal.

The body position estimation module 112 estimates the user's body position during sleep, based on the acceleration signals (x-axis acceleration signal, y-axis acceleration signal, and z-axis acceleration signal) detected by the acceleration signal detector 111. The user's body position estimated by the body position estimation module 112 includes, for example, a supine position, a prone position, a lateral position, etc.

The respiratory signal detector 113 is a functional module implemented by the high-pass filter 107b and the amplifier circuit 107c. The respiratory signal detector 113 cuts the direct-current component of each acceleration signal detected by the acceleration signal detector 111, by the high-pass filter 107b and extracts the alternating-current component (high-frequency component) of the acceleration signal. In addition, the respiratory signal detector 113 amplifies the extracted alternating-current component of the acceleration signal by the amplifier circuit 107c. The respiratory signal detector 113 thereby detects the amplified alternating-current component of the acceleration signal as the signal (second signal) for estimation of a state (second state) other than the user's body position. In the following explanations, the signal detected by the respiratory signal detector 113 (i.e., the amplified alternating-current component of the acceleration signal) is called the respiratory signal.

The respiratory state estimation module 114 estimates the user's respiratory state during sleep, based on the acceleration signals detected by the acceleration signal detector 111 and the respiratory signals detected by the respiratory signal detector 113. The user's respiratory state estimated by the respiratory state estimation module 114 includes, for example, presence or absence of a symptom of snoring during sleep, etc.

Next, an operation of the sleep state estimation device 10 attached to the user's chest, etc., to estimate the user's sleep state will be explained.

In the present embodiment, when the acceleration signals (x-axis acceleration signal, y-axis acceleration signal and z-axis acceleration signal) are detected by the acceleration signal detector 111 (acceleration sensor 107a), processing of estimating the user's body position during sleep (hereinafter called body position estimation processing) and processing of estimating the user's respiratory state during sleep (hereinafter called respiratory state estimation processing) as the user's sleep state are executed. The body position estimation processing and the respiratory state estimation processing will be hereinafter explained. The body position estimation processing and the respiratory state estimation processing are assumed to be periodically executed during the user's sleep period.

First, a procedure of the body position estimation processing will be explained with reference to a flowchart of FIG. 8.

In the body position estimation processing, the acceleration signals detected by the acceleration signal detector 111 are converted into digital signals by the A/D converter 107d (step S1). The acceleration signals thus converted into the digital signals are supplied to the body position estimation module 112.

Next, the body position estimation module 112 estimates the user's body position during sleep, based on the acceleration signals converted into the digital signals by the A/D converter 107d (step S2).

The processing of step S2 will be explained more specifically. Each of the acceleration signals (x-axis acceleration signal, y-axis acceleration signal, and z-axis acceleration signal) detected by the acceleration signal detector 111 includes the direct-current component (gravitational acceleration), and the direction of the gravitational acceleration to the sleep state estimation device 10 can be computed from (the direct-current component of) each of the acceleration signals. The user's body position is estimated based on the direction of the gravitational acceleration to the sleep state estimation device 10 thus computed.

More specifically, when the direction of the gravitational acceleration to the sleep state estimation device 10 is a user's backward direction on the z-axis (i.e., user's back surface direction), the user's body position is assumed to be the supine position. In contrast, when the direction of the gravitational acceleration to the sleep state estimation device 10 is a user's forward direction on the z-axis (i.e., user's front surface direction), the user's body position is assumed to be the prone position. In addition, when the direction of the gravitational acceleration to the sleep state estimation device 10 is the x-axis direction (i.e., user's lateral direction), the user's body position is assumed to be the lateral position.

When the user's body position is estimated in step S2, the body position estimation module 112 outputs the estimation result to an external Bluetooth-enabled device (smartphone, tablet computer, PC, etc.) via, for example, the BT module 108, etc. (step S3). The estimation result thus output to the Bluetooth-enabled device is provided (presented) to, for example, the user, etc., by the device.

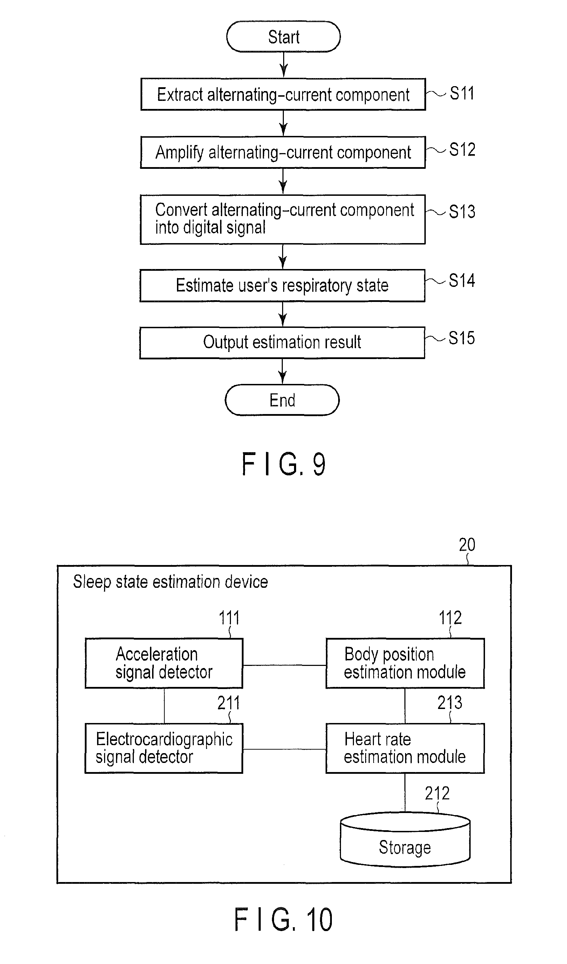

Next, a procedure of the respiratory state estimation processing will be explained with reference to a flowchart of FIG. 9.

In the respiratory state estimation processing, the respiratory signal detector 113 executes filtering using the high-pass filter 107b, for each of the acceleration signals detected by the acceleration signal detector 111. According to this processing, the respiratory signal detector 113 extracts the alternating-current component of the x-axis acceleration signal, the alternating-current component of the y-axis acceleration signal, and the alternating-current component of the z-axis acceleration signal, from the respective acceleration signals (x-axis acceleration signal, y-axis acceleration signal, and z-axis acceleration signal) detected by the acceleration signal detector 111 (step S11).

The A/D converter 107d in the present embodiment needs to A/D-convert both (the direct-current component of) each of the acceleration signals detected by the acceleration signal detector 111 and the alternating-current component of the acceleration signal, to estimate the user's body position and the respiratory state. In this case, the alternating-current component of each of the acceleration signals is extremely smaller in amplitude than the acceleration signal. More specifically, detection of the acceleration signal within a range of ±1G is required for estimation of the body position, but the magnitude of the acceleration signal detected for estimation of the respiratory state is, for example, several milliG or smaller. In contrast, resolution of the A/D converter 107d which can be mounted on the sleep state estimation device 10 is, for example, approximately 10 bit.

In other words, the alternating-current component of each of the acceleration signals extracted by using the high-pass filter 107b cannot be processed by the A/D converter 107d as it is. Thus, the respiratory signal detector 113 amplifies the extracted alternating-current component of each of the acceleration signals, by the amplifier circuit 107c (step S12). In this case, an amplification rate of the alternating-current component of each of the acceleration signals is assumed to be adjusted in accordance with a dynamic range of the A/D converter 107d such that the alternating-current component can be processed even by the resolution of the A/D converter 107d.

The alternating-current component of each of the acceleration signals thus amplified by the respiratory signal detector 113 (i.e., the respiratory signal detected by the respiratory signal detector 113) is converted into a digital signal by the A/D converter 107d (step S13). The respiratory signal converted into the digital signal is supplied to the respiratory state estimation module 114.

Next, the respiratory state estimation module 114 estimates the user's respiratory state (presence or absence of a symptom of snoring) during sleep, based on the respiratory signals (alternating-current components of the acceleration signals) converted into the digital signals by the A/D converter 107d (step S14).

The processing in step S14 will be explained more specifically. In this case, the respiratory state estimation module 114 estimates the presence or absence of a symptom of snoring based on, for example, a variable computed based on the respiratory signal.

A variable (Y) to thus estimate the presence or absence of a symptom of snoring is computed by, for example, an expression such as "Kx(Axac)²+Ky(Ayac)²+Kz(Azac)²".

In this expression, Axac indicates the alternating-current component of the x-axis acceleration signal. Similarly, Ayac and Azac indicate the alternating-current component of the y-axis acceleration signal and the alternating-current component of the z-axis acceleration signal, respectively. Kx, Ky and Kz indicate values representing weights on the x-axis acceleration signal (Axac), the y-axis acceleration signal (Ayac) and the z-axis acceleration signal (Azac), respectively. In other words, the variable to estimate the presence or absence of a symptom of snoring is computed based on the alternating-current component of the x-axis acceleration signal, the alternating-current component of the y-axis acceleration signal, and the alternating-current component of the z-axis acceleration signal, which are weighted by Kx, Ky and Kz, respectively.

When the sleep state estimation device 10 is attached to the user by the electrically conductive gel 14, vibration may be damped, and a main axis of the vibration may be changed according to the directions of the gravitational accelerations, since the gel 14 is thick and elastic. Thus, in the present embodiment, the weights (i.e., Kx, Ky and Kz) on the alternating-current component of the x-axis acceleration signal, the alternating-current component of the y-axis acceleration signal, and the alternating-current component of the z-axis acceleration signal, are varied in response to the gravitational accelerations (direct-current components of the respective acceleration signals).

In this case, magnitudes of Kx, Ky and Kz are set to be varied in proportion to the direct-current component Axdc of the x-axis acceleration signal, the direct-current component Aydc of the y-axis acceleration signal, and the direct-current component Azdc of the alternating current component of the z-axis acceleration signal, respectively. In other words, a ratio of Kx, Ky and Kz is varied in accordance with a ratio of the direct-current component Axdc of the x-axis acceleration signal, the direct-current component Aydc of the y-axis acceleration signal, and the direct-current component Azdc of the z-axis acceleration signal. Alternatively, a relationship among Kx, Ky and Kz or a relationship among Axdc, Aydc, and Azdc may not be a simply proportional relationship, but may be determined by a nonlinear function.

The direct-current component Axdc of the x-axis acceleration signal, the direct-current component Aydc of the y-axis acceleration signal, and the direct-current component Azdc of the z-axis acceleration signal (i.e., low frequency components) are assumed to be extracted by, for example, a low-pass filter, etc. In addition, a function for uniquely determining Kx, Ky and Kz according to the direct-current component Axdc of the x-axis acceleration signal, the direct-current component Aydc of the y-axis acceleration signal, and the direct-current component Azdc of the z-axis acceleration signal is assumed to be prestored in, for example, the sleep state estimation device 10.

In step S14, when the variable computed as explained above is equal to or greater than a predetermined value, it can be estimated that the user has a symptom of snoring.

When the user's respiratory state is thus estimated, the respiratory state estimation module 114 outputs (transmits) the estimation result to an external Bluetooth-enabled device via, for example, the above-explained BT module 108, etc. (step S15). The estimation result thus output to the Bluetooth-enabled device is provided (presented) to, for example, the user, etc., by the device. The estimation result output in step S15 may include, for example, a degree of snoring caused by a symptom of snoring besides the presence or absence of the symptom of snoring.

When the above-explained respiratory state estimation processing is executed, Kx, Ky and Kz determined in step S14 are assumed to be held in, for example, the sleep state estimation device 10. According to this, when the respiratory state estimation processing is executed again but the user's body position is not changed, the user's respiratory state can be estimated by using Kx, Ky and Kz held in the sleep state estimation device 10, without determining Kx, Ky and Kz again.

The body position estimation processing and the respiratory state estimation processing have been mainly explained in the present embodiment, but the user's heart rate can also be estimated by using the electrocardiographic sensor module 106 (electrocardiographic sensor 106a) in the sleep state estimation device 10 of the present embodiment. Furthermore, a respiratory rate, etc., can also be estimated by connecting a sensor configured to sense impedance to the electrocardiographic electrodes 11 and 12 and sensing variation in the impedance between the electrodes by the sensor.

In the present embodiment, as explained above, the acceleration signals representing the acceleration acting on the sleep state estimation device 10 are detected and the user's body position is estimated based on the detected acceleration signals (gravitational accelerations). Moreover, in the present embodiment, the signal (respiration signal) to estimate the user's respiratory state (state other than the user's body position) is detected, and the user's respiratory state is estimated based on the detected acceleration signals and the respiration signal. More specifically, in the present embodiment, the alternating-current components of the acceleration signals are weighted according to the direct-current components of the acceleration signals in the directions of the three orthogonal axes, respectively, and the user's respiratory state (presence or absence of a symptom of snoring) is estimated based on the weighted alternating-current components of the acceleration signals. In other words, in the present embodiment, the acceleration signals (gravitational accelerations) which are the signals to estimate the user's body position are used when the presence or absence of a symptom of snoring is estimated (or the variable used for the estimation is computed), and a processing method (weight value) for estimating the user's respiratory state is varied according to the directions of the gravitational accelerations (i.e., user's body position).

In the present embodiment, since influence from the elasticity of the electrically conductive gel 14 and the gravitational accelerations can be excluded and the sensitivity in vibration detection can be highly maintained by the structure, accuracy in estimation of the user's sleep state (presence or absence of a symptom of snoring) can be improved.

In addition, in the present embodiment, since the user's respiratory state is estimated by using the alternating-current components extracted from the acceleration signals detected by the acceleration signal detector 111, sensors for estimating the user's body position and the user's respiratory state do not need to be provided separately. In the present embodiment, the miniaturization, reduction of the weight, and thinning of the sleep state estimation device 10 can be thereby implemented.

Moreover, in the present embodiment, since processing can be executed appropriately with the A/D converter 107d of low resolution by amplifying the alternating-current components extracted from the acceleration signals detected by the acceleration signal detector 111, an expensive A/D converter 107d does not need to be employed and reduction of manufacturing costs can be implemented.

Use of the sleep state estimation device 10 attached to the user's chest is explained in the present embodiment but, for example, if the user's body position and the user's respiratory state during sleep can be estimated with the acceleration signals sensed by the acceleration sensor 107a, the sleep state estimation device 10 may be attached to the other site.

In addition, outputting the estimation result on the user's body position and the user's respiratory state to the Bluetooth-enabled device has been explained in the present embodiment, but the estimation result may be output to, for example, an external server device, etc., which functions as a cloud server providing cloud computing service over a wireless LAN, etc., and stored in the server device. Alternatively, the estimation result may be stored in the sleep state estimation device 10.

Moreover, the sleep state estimation device 10 including the body position estimation module 112 and the respiratory state estimation module 114 is explained in the present embodiment, but the processing executed by the body position estimation module 112 and the respiratory state estimation module 114 may be executed by, for example, an external Bluetooth-enabled device, a server device or the like.

### (Second Embodiment)

Next, a second embodiment will be described.

The present embodiment is different from the First Embodiment with respect to a feature of estimating the user's heart rate during sleep by using the user's body position estimated based on an acceleration signal. In other words, the user's sleep state estimated by a sleep state estimation device of the present embodiment includes, for example, a body position and a heart rate of the user during sleep.

FIG. 10 is a block diagram mainly showing a functional configuration of the sleep state estimation device 20 of the present embodiment. In FIG. 10, portions like or similar to those shown in FIG. 7 are denoted by the same reference numbers and symbols, and detailed explanations are omitted. Portions different from FIG. 7 will be mainly explained. Since the system configuration of the sleep state estimation device 20 of the present embodiment is the same as that of the First Embodiment, the device will be explained as needed with reference to FIG. 4 to FIG. 6, etc.

The sleep state estimation device 20 includes an electrocardiographic signal detector 211, a storage 212 and a heart rate estimation module 213 besides the acceleration signal detector 111 and the body position estimation module 112 of the First Embodiment, as shown in FIG. 10.

The electrocardiographic signal detector 211 is a functional module implemented by the electrocardiographic sensor 106a, and detects the electrocardiographic signal by the electrocardiographic sensor 106a.

The storage module 212 stores information on a plurality of processing methods (contents) for estimating the user's heart rate, based on the electrocardiographic signal detected by the electrocardiographic signal detector 211. In other words, the processing methods corresponding to the respective user's body positions estimated by the body position estimation module 112 are defined in the storage 212. As the plurality of processing methods (contents), for example, a plurality of arbitrary, publicly known methods such as simple peak detection, Pans & Tompkins and pattern matching can be employed. Alternatively, a plurality of parameters (for example, threshold values) used in the at least one of the methods may be prepared. In addition, correspondence between the plurality of processing methods (contents) and the user's body positions may be varied in accordance with each of user's characteristics (orientation of a heart, composition of chest tissues, etc., different in individual persons).

The heart rate estimation module 213 determines (selects) the processing method corresponding to the user's body position estimated by the body position estimation module 112, of the plurality of processing methods defined in the storage 212. The heart rate estimation module 213 estimates the user's heart rate by executing processing based on the determined processing method for the electrocardiographic signal detected by the electrocardiographic signal detector 211.

Next, an operation of the sleep state estimation device 20 attached to the user's chest, etc., to estimate the user's sleep state will be explained.

In the present embodiment, when acceleration signals are detected by the acceleration signal detector 111 (acceleration sensor 107a), the processing of estimating the user's body position shown in FIG. 8 is executed, similarly to the First Embodiment. Furthermore, processing of estimating the user's heart rate during sleep (hereinafter referred to as heart rate estimation processing) is executed besides body position estimation processing, in the present embodiment. The heart rate estimation processing is assumed to be periodically executed during the user's sleep period, similarly to the above-explained body position estimation processing.

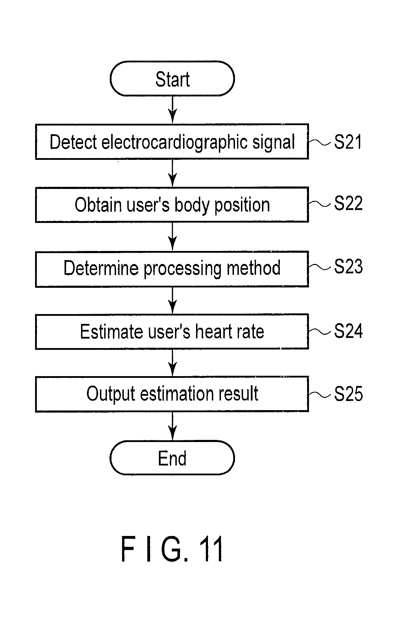

Next, a procedure of the heart rate estimation processing will be explained with reference to a flowchart of FIG. 11.

In the heart rate estimation processing, the electrocardiographic signal detector 211 detects the electrocardiographic signal indicating electrocardiogram of the user to which the sleep state estimation device 20 is attached, by using the electrocardiographic sensor 106a (step S21).

When the electrocardiographic signal is thus detected by the electrocardiographic signal detector 211, the heart rate estimation module 213 obtains the user's body position estimated by the body position estimation processing, from the body position estimation module 112 (step S22).

When a small sensor having a short distance between the electrocardiographic electrodes such as the sleep state estimation device 20 of the present embodiment is attached to a chest, a waveform of the electrocardiogram obtained from the electrocardiographic signal (electrocardiographic waveform) is greatly varied according to the user's body position (attitude). In addition, the heart rate is estimated from the electrocardiographic signal (waveform of the electrocardiogram) in the present embodiment, but an optimum processing method (algorithms, parameters, etc.) for estimating the heart rate is changed depending on the waveform of the electrocardiogram. Thus, in the present embodiment, the processing method for estimating the heart rate (estimation method) is changed in accordance with the estimated user's body position.

In this case, the heart rate estimation module 213 determines the processing method corresponding to the user's body position obtained in step S22 (i.e., processing method for appropriately processing the waveform of the electrocardiogram estimated to be obtained in accordance with the user's body position), by referring to the storage 212 (step S23).

Next, the heart rate estimation module 213 executes the processing based on the processing method determined in step S23 (i.e., processing corresponding to the estimated user's body position), based on the electrocardiographic signal detected by the electrocardiographic signal detector 211. According to this, for example, a time-series heart rate is computed and the user's heart rate is estimated (step S24).

When the user's heart rate is thus estimated, the heart rate estimation module 213 outputs the estimation result to, for example, an external Bluetooth-enabled device, a server device, or the like (step S25).

When the above-explained heart rate estimation processing is executed, the processing method determined in step S23 is assumed to be held in, for example, the sleep state estimation device 10. According to this, when the heart rate estimation processing is executed again but the user's body position is not changed, the user's heart rate can be estimated based on the processing method held in the sleep state estimation device 10, without determining the processing method again.

In the present embodiment, as explained above, the user's heart rate is estimated by detecting the electrocardiographic signal indicating the user's electrocardiogram as the signal for estimating the user's heart rate and by executing the processing corresponding to the user's body position estimated based on the acceleration signals. In other words, in the present embodiment, accuracy in estimation of the user's sleep state (heart rate) can be enhanced since the heart rate can be estimated by using the optimum algorithm, parameter, etc., corresponding to the user's body position by changing the processing method for estimating the heart rate in accordance with the user's body position.

The present embodiment is explained on the assumption that the processing method for estimating the user's heart rate is uniquely determined in accordance with the user's body position, but the processing method often cannot be uniquely determined in accordance with the user's body position (i.e., a plurality of processing methods correspond to the user's body position). In this case, all types of processing are executed in a plurality of processing methods according to the user's body position, and the processing method which obtains a most desirable processing result of all the processing results is adopted. The time-series heart rate is computed as the processing result in the present embodiment, but desirability of the processing result is evaluated based on, for example, whether the computed heart rate falls within a predetermined range or not (for example, a rate of a value which falls within the range), etc. In this structure, the user's heart rate can be estimated by using the optimum algorithm, parameter, etc., even if the processing method cannot be uniquely determined based on the user's body position.

In addition, in the present embodiment, the acceleration sensor module 107 may not includes the high-pass filter 107b or the amplifier circuit 107c since the acceleration signal is used for estimation of the user's body position alone and the alternating-current component does not need to be extracted from the acceleration signal.

The acceleration sensor 107a outputs the analog signal in the First Embodiment, but the acceleration sensor 107a of the present embodiment may output the digital signal since the acceleration signal is used for estimation of the user's body position alone in the present embodiment.

Use of the sleep state estimation device 20 attached to the user's chest is explained in the present embodiment, but the sleep state estimation device 20 may be attached to the other site if, for example, the user's body position during sleep can be estimated with the acceleration signals sensed by the acceleration sensor 107a and the user's heart rate during sleep can be estimated with the electrocardiographic signal sensed by the electrocardiographic sensor 106a.

Moreover, the sleep state estimation device 20 including the body position estimation module 112 and the heart rate estimation module 213 is explained in the present embodiment, but the processing executed by the body position estimation module 112 and the heart rate estimation module 213 may be executed by, for example, an external Bluetooth-enabled device, a server device or the like.

### (Third Embodiment)

Next, a third embodiment will be described. The present embodiment is different from the First Embodiment with respect to a feature of using a microphone to estimate a user's respiratory state (presence or absence of a symptom of snoring).

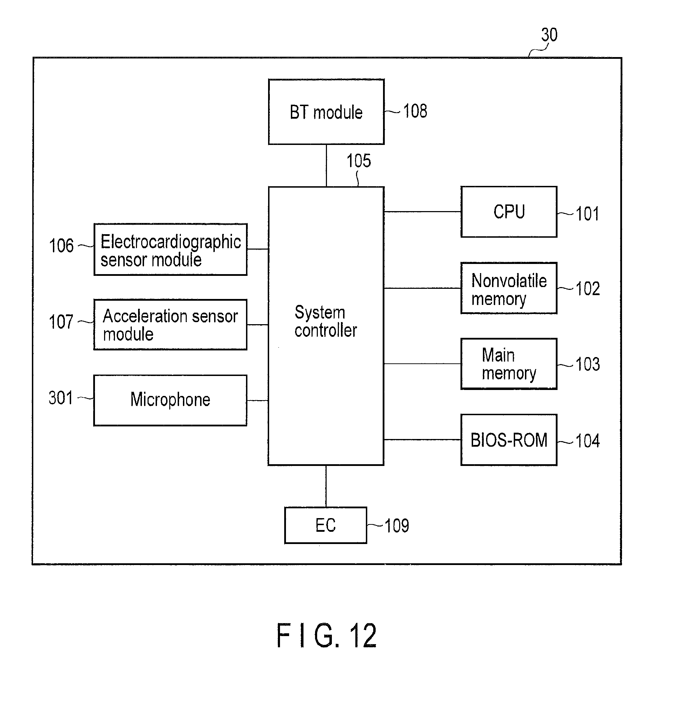

FIG. 12 is a block diagram mainly showing a functional configuration of the sleep state estimation device 30 of the present embodiment. In FIG. 12, portions like or similar to those shown in FIG. 4 are denoted by the same reference numbers and symbols, and detailed explanations are omitted. Portions different from FIG. 4 will be mainly explained.

The sleep state estimation device 30 further includes a microphone 301 besides the system configuration of the First Embodiment, as shown in FIG. 12.

The microphone 301 is used to estimate the user's respiratory state during sleep, and senses a sound signal produced in response to the user's respiratory state. According to the microphone 301, sound around the sleep state estimation device 30 can be converted into an electrical signal (sound signal).

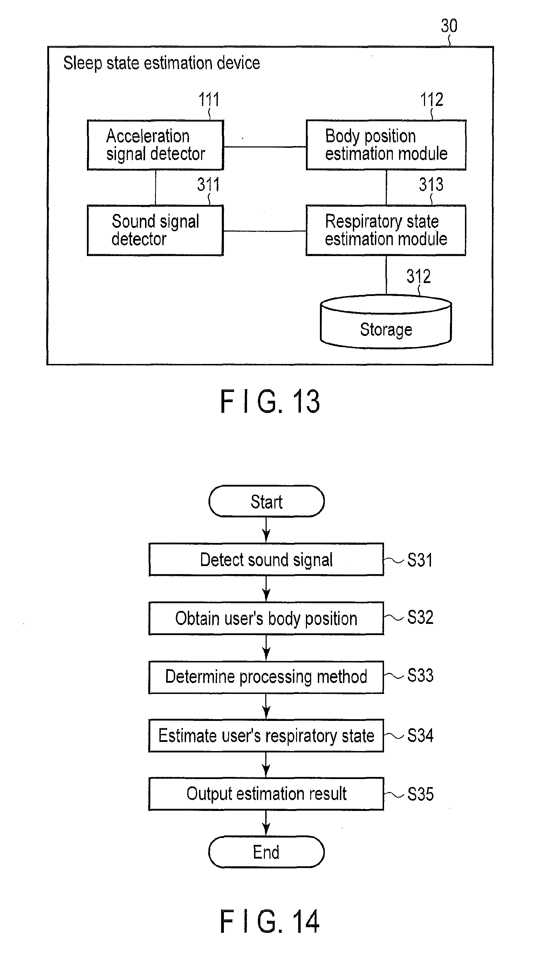

FIG. 13 is a block diagram mainly showing a functional configuration of the sleep state estimation device 30 of the present embodiment. In FIG. 13, portions like or similar to those shown in FIG. 7 are denoted by the same reference numbers and symbols, and detailed explanations are omitted. Portions different from FIG. 7 will be mainly explained.

The sleep state estimation device 30 includes a sound signal detector 311, a storage 312 and a respiratory state estimation module 313 besides the acceleration signal detector 111 and the body position estimation module 112 of the First Embodiment, as shown in FIG. 13.

The sound signal detector 311 is a functional module implemented by the microphone 301, and detects the sound signal by using the microphone 301. The sound signal detected by the sound signal detector 311 includes, for example, a signal of a respiratory sound produced by the user's symptom of snoring during sleep (hereinafter called a snoring signal), other noise or the like as the sound signal produced in response to the user's respiratory state.

The storage 312 stores information on a plurality of processing methods (contents) for estimating the user's respiratory rate (for example, presence or absence of a symptom of snoring), based on the sound signal detected by the sound signal detector 311. In other words, the processing methods corresponding to the respective user's body positions estimated by the body position estimation module 112 are defined in the storage 312. The processing method defined in the storage 312 includes, for example, a processing method for removing noise from the sound signal detected by the sound signal detector 311, or the like. As the plurality of processing methods (contents) for removing the noise, for example, a plurality of arbitrary, publicly known techniques employed for removal of noise from an acoustic signal, such as an infinite impulse response (IIR) filter, an adaptive filter and nonnegative matrix factorization (NMF) can be employed. Alternatively, a plurality of parameters (for example, filter coefficients) used in the at least one of the methods may be prepared.

In addition, correspondence between the plurality of processing methods (contents) and the user's body positions may be varied in accordance with a positional relationship with a noise source in an environment in which the sleep state estimation device 30 is used. More specifically, for example, in an environment in which a TV is located on a user's right side and an air-conditioner is located on a user's left side, a processing method suitable for removing noise from the TV is employed when the user's body position is a right-side lateral position while a processing method suitable for removing noise from the air-conditioner is employed when the user's body position is a left-side lateral position.

The respiratory state estimation module 313 determines (selects) the processing method corresponding to the user's body position as estimated by the body position estimation module 112, of the plurality of processing methods defined in the storage module 312. The respiratory state estimation module 313 estimates the user's respiratory state by executing processing (for example, noise removing processing) based on the determined processing method for the sound signal detected by the sound signal detector 311.

Next, an operation of the sleep state estimation device 30 attached to the user's chest, etc., to estimate the user's sleep state will be explained.

In the present embodiment, when acceleration signals are detected by the acceleration signal detector 111 (acceleration sensor 107a), the processing of estimating the user's body position shown in FIG. 8 is executed, similarly to the First Embodiment. Furthermore, processing of estimating the user's respiratory state during sleep (hereinafter referred to as respiratory state estimation processing) is executed besides body position estimation processing, in the present embodiment. The respiratory state estimation processing is assumed to be periodically executed during the user's sleep period, similarly to the above-explained body position estimation processing.

Next, a procedure of the respiratory state estimation processing of the present embodiment will be explained with reference to a flowchart of FIG. 14.

In the respiratory state estimation processing, the sound signal detector 311 detects the sound signal indicating the sound around the sleep state estimation device 30 by using the microphone 301 (step S31).

When the sound signal is thus detected by the sound signal detector 311, the respiratory state estimation module 313 obtains the user's body position estimated by the body position estimation processing, from the body position estimation module 112 (step S32).

When the sound signal is detected by using the microphone 301 in the sleep state estimation device 30 of the present embodiment, the type of the noise included in the sound signal is changed by change in the orientation of the sleep state estimation device 30. In the present embodiment, the respiratory state is estimated from the sound signal in the present embodiment, but an optimum processing method (algorithms, parameters, etc.) for estimating the respiratory state is changed when the type of the noise included in the sound signal is changed. Thus, in the present embodiment, the processing method for removing the noise included in the sound signal is changed in accordance with the estimated user's body position.

In this case, the respiratory state estimation module 313 determines the processing method corresponding to the user's body position obtained in step S32 (i.e., processing method for appropriately removing the noise estimated to be included in the sound signal in accordance with the user's body position), by referring to the storage 312 (step S33).

More specifically, for example, when the TV is located on the user's right side as explained above, and when the user's body position is the right-side lateral position, the sound signal detected by the sound signal detector 311 may include noise caused by sound from the TV besides the snoring signal. In this case, the processing method for removing the noise caused by the sound from the TV is determined as the processing method corresponding to the user's body position.

Next, the respiratory state estimation module 313 removes the noise by executing processing (noise removing processing) based on the determined processing method for the sound signal detected by the sound signal detector 311, and estimates the user's respiratory state based on the sound signal from which the noise is removed (step S34). In this case, when the sound signal from which the noise is removed is analyzed and the sound signal is determined to include the snoring signal, it can be estimated that the user has the symptom of snoring.

When the user's respiratory status is thus estimated, the respiratory state estimation module 313 outputs the estimation result to, for example, an external Bluetooth-enabled device, a server device, or the like (step S35).

Presence or absence of a symptom of snoring alone is explained for convenience but, for example, the degree of snoring, etc., in the symptom of snoring may be included as the estimation result on the user's respiratory state, as explained in the First Embodiment.

When the above-explained respiratory state estimation processing is executed, the processing method determined in step S33 is assumed to be held in, for example, the sleep state estimation device 30. According to this, when the respiratory state estimation processing is executed again but the user's body position is not changed, the user's respiratory state can be estimated based on the processing method held in the sleep state estimation device 30, without determining the processing method again.

In the present embodiment, as explained above, the user's respiratory state (presence or absence of a symptom of snoring) is estimated by detecting the sound signal produced in response to the user's respiratory state as the signal for estimating the user's respiratory state and by executing the processing corresponding to the user's body position estimated based on the acceleration signals. In other words, in the present embodiment, accuracy in estimation of the user's sleep state (respiratory state) can be enhanced since the respiratory state can be estimated by using the optimum algorithm, parameter, etc., corresponding to the user's body position by varying the processing method for estimating the respiratory state in accordance with the user's body position.

The present embodiment is explained on the assumption that the processing method for estimating the user's respiratory state is uniquely determined in accordance with the user's body position, but the processing method often cannot be uniquely determined in accordance with the user's body position (i.e., a plurality of processing methods correspond to the user's body position). In this case, all types of processing (noise removing processing) are executed in a plurality of processing methods according to the user's body position, and the processing method which obtains a most desirable processing result of all the processing results is adopted, as explained in the Second Embodiment. The desirability of the processing result in this case is evaluated based on, for example, whether the shape of the noise-removed frequency spectrum falls within a predetermined range or not, etc. In this structure, the user's respiratory state can be estimated by using the optimum algorithm, parameter, etc. even if the processing method cannot be uniquely determined based on the user's body position.

In addition, in the present embodiment, the acceleration sensor module 107 may not includes the high-pass filter 107b or the amplifier circuit 107c since the alternating-current component does not need to be extracted from the acceleration signal. In addition, the acceleration sensor 107a of the present embodiment may output the analog signal or may output the digital signal, similarly to the Second Embodiment.

Use of the sleep state estimation device 30 attached to the user's chest is explained in the present embodiment, but the sleep state estimation device 30 may be attached to the other site if, for example, the user's body position during sleep can be estimated with the acceleration signals sensed by the acceleration sensor 107a and the user's respiratory state during sleep can be estimated with the sound signal sensed by the microphone 301.

Moreover, the sleep state estimation device 30 including the body position estimation module 112 and the respiratory state estimation module 313 is explained in the present embodiment, but the processing executed by the body position estimation module 112 and the respiratory state estimation module 313 may be executed by, for example, an external Bluetooth-enabled device, a server device or the like.

The user's respiratory state or heart rate is estimated as the user's state other than the body position in each of the above-explained embodiments but, for example, if the estimation accuracy can be enhanced by using the acceleration signals (or estimation results of user's body positions) sensed by the acceleration sensor 107a, each of the embodiments may be applied to a case where a state other than these states is estimated. In addition, the electrocardiographic sensor 106a, the acceleration sensor 107a and the microphone 301 are used in each of the above-explained embodiments but, if the accuracy in estimation of the user's respiratory state can be enhanced by combining (signals detected by) a plurality of sensors, each of the embodiments may be applied to a case of using a sensor other than these sensors.

In each of the above-explained embodiments, the sleep state estimation device is attached by the electrically conductive gel 14 having elasticity, but the sleep state estimation device may be attached to the user in the other methods.

Since the processing of each of the above-explained embodiments can be implemented by a computer program, the same advantage as those of the embodiments can easily achieved by installing the computer program in a computer and executing the computer program.

According to at least one of the above-explained embodiments, a sleep state estimation device, a method and a storage medium capable of enhancing the accuracy in estimation of the user's respiratory state can be provided.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A sleep state estimation device (10) attached to a user during sleep and used, **characterized by** comprising:
a first detector (111) configured to detect a first signal to estimate a first state of the user;
a first estimation module (112) configured to estimate the user's first state, based on the first signal;
a second detector (113) configured to detect a second signal to estimate a second state of the user other than the user's first state; and
a second estimation module (114) configured to estimate the user's second state by using the second signal, based on an estimation method determined in accordance with the first signal.

2. The device of Claim 1, **characterized in that**
the first detector is further configured to detect an acceleration signal indicating acceleration acting on the device, and
the first estimation module is further configured to estimate a body position of the user, based on the acceleration signal.

3. The device of Claim 2, **characterized in that**
the first detector is further configured to detect first to third acceleration signals indicating accelerations in directions of first to third axes orthogonal to each other, respectively,
the second detector is further configured to detect alternating-current components of the first to third acceleration signals from the respective first to third acceleration signals, and
the second estimation module is further configured to weight the alternating-current components of the first to third acceleration signals in accordance with direct-current components of the first to third acceleration signals, respectively, and estimate a respiratory state of the user, based on the weighted alternating-current components of the first to third acceleration signals.

4. The device of Claim 3, **characterized in that**
the second detector includes an amplifier (107c) configured to amplify each of the alternating-current components of the first to third acceleration signals.

5. The device of Claim 3, **characterized by** further comprising
an electrically conductive material (14) having elasticity which allows the device to be attached to the user.

6. The device of Claim 2, **characterized in that**
the second detector (211) is further configured to detect an electrocardiographic signal indicating electrocardiogram of the user, and
the second estimation module (213) is further configured to estimate a heart rate of the user by executing a processing corresponding to the user's body position estimated based on the acceleration signal, of a plurality of processing for estimating the user's heart rate, based on the electrocardiographic signal.

7. The device of Claim 2, **characterized in that**
the second detector (311) is further configured to detect a sound signal produced in accordance with a respiratory state of the user, and
the second estimation module (313) is further configured to estimate the user's respiratory state by executing a processing corresponding to the user's body position estimated based on the acceleration signal, of a plurality of processing for estimating the user's respiratory state, based on the sound signal.

8. A sleep state estimation method executed by a sleep state estimation device (10) attached to a user during sleep and used, the method **characterized by** comprising:
detecting a first signal to estimate a first state of the user;
estimating the user's first state, based on the first signal;
detecting a second signal to estimate a second state of the user other than the user's first state; and
estimating the user's second state by using the second signal, based on an estimation method determined in accordance with the first signal.

9. A non-transitory computer-readable storage medium having stored thereon a computer program which is executable by a computer of a sleep state estimation device (10) comprising a first detector configured to detect a first signal to estimate a first state of a user during sleep, and a second detector configured to detect a second signal to estimate a second state of the user other than the user's first state, the computer program **characterized by** comprising instructions capable of causing the computer to execute functions of:
estimating the user's first state, based on the first signal; and
estimating the user's second state by using the second signal, based on an estimation method determined in accordance with the first signal.
